**Europäisches Patentamt**

**European Patent Office**

**Office européen des brevets**

(19)

(11) Veröffentlichungsnummer : **0 352 641 B1**

(12) # EUROPÄISCHE PATENTSCHRIFT

(45) Veröffentlichungstag der Patentschrift :
**16.09.92 Patentblatt 92/38**

(51) Int. Cl.$^5$ : **C07C 67/38, C07C 69/533**

(21) Anmeldenummer : **89113381.1**

(22) Anmeldetag : **21.07.89**

(54) **Verfahren zur Herstellung von Pentensäurealkylestern.**

(30) Priorität : **26.07.88 DE 3825296**

(43) Veröffentlichungstag der Anmeldung :
**31.01.90 Patentblatt 90/05**

(45) Bekanntmachung des Hinweises auf die
Patenterteilung :
**16.09.92 Patentblatt 92/38**

(84) Benannte Vertragsstaaten :
**BE CH DE ES FR GB IT LI NL**

(56) Entgegenhaltungen :
**EP-A- 0 031 100**

(56) Entgegenhaltungen :
**EP-A- 0 042 928**
**DE-A- 2 837 815**
**US-A- 3 778 466**
**PATENT ABSTRACTS OF JAPAN, Band 2, Nr.
59 (C-78)(375), 27. April 1978; & JP-A-53 15309**

(73) Patentinhaber : **BASF Aktiengesellschaft
Carl-Bosch-Strasse 38
W-6700 Ludwigshafen (DE)**

(72) Erfinder : **Maerkl, Robert, Dr.
Bolander Weg 23
W-6701 Fussgoenheim (DE)**
Erfinder : **Harder, Wolfgang, Dr.
Bergwaldstrasse 16
W-6940 Weinheim (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der US-Patentschrift 4 550 195 ist ein Verfahren zur Herstellung von Pentensäurealkylestern bekannt, bei dem man Butadien enthaltende $C_4$-Schnitte mit Kohlenmonoxid und Alkanolen in Gegenwart von Kobaltcarbonylkatalysatoren und heterocyclischen aromatischen tertiären Stickstoffbasen umsetzt. Bei der technischen Durchführung des Verfahrens war es erwünscht, den Katalysator wieder zu verwenden. Es hat sich jedoch herausgestellt, daß hierbei die Raum-Zeit-Ausbeute zu wünschen übrig läßt.

Auch nach dem in Bulletin of the Chemical Society of Japan, Band 46 (1973), Seite 125 beschriebenen Verfahren zur Herstellung von Pentensäureestern durch Carbalkoxylieren von Butadien sinkt bei der Wiederverwendung des Kobaltkatalysators der Umsatz an Butadien fortwährend ab, während der Anteil an Nebenprodukten ansteigt.

Nachdem in der europäischen Patentschrift 10 581 beschriebenen Verfahren wird das bei der Pentensäurealkylesterherstellung erhaltene Reaktionsgemisch abgekühlt, in zwei Phasen getrennt und die Kobaltkatalysator enthaltende Phase partiell zurückgeführt. Es hat sich jedoch herausgestellt, daß die Phasentrennung Schwierigkeiten bereitet. Zudem enthält die den Kobaltkatalysator enthaltende Phase zusätzlich bis zu 30 % Pentenester, der wiederum zurückgeführt wird und weiteren Umsetzungen zugänglich ist.

Es war deshalb die technische Aufgabe gestellt, bei der Herstellung von Pentensäurealkylestern durch Carbalkoxylierung von Butadien unter Rückführung des Katalysators und unter Einhaltung einer hohen Selektivität und eines hohen Umsatzes die Raum-Zeit-Ausbeute an Pentensäurealkylestern zu steigern, ohne daß die Bildung von Nebenprodukten ansteigt.

Diese Aufgabe wird gelöst in einem Verfahren zur Herstellung von Pentensäurealkylestern, wobei man
a) Butadien oder Butadien enthaltende Kohlenwasserstoffgemische mit Kohlenmonoxid und Alkanolen in Gegenwart von tertiären aromatischen heterocyclischen Stickstoffbasen und Kobaltcarbonylkatalysatoren bei einer Temperatur von 80 bis 160°C und unter einem Druck von 100 bis 1.000 bar umsetzt und ein Pentensäurealkylester, $C_6$-Diester, tertiäre Stickstoffbasen, Alkanole, Butadien, Kohlenwasserstoffe und Nebenprodukte enthaltendes Reaktionsgemisch erhält
b) aus dem erhaltenen Reaktionsgemisch überschüssige Kohlenwasserstoffe, Stickstoffbasen, Alkanole sowie Pentensäurealkylester durch Destillation weitgehend abtrennt und einen Kobaltcarbonylkatalysator enthaltenden Rückstand erhält
c) den Kobaltcarbonylkatalysator enthaltenden Rückstand bei erhöhter Temperatur und unter erhöhtem Druck mit einem Gemisch aus Kohlenmonoxid und Wasserstoff in Berührung bringt
d) den Kobaltcarbonylkatalysator enthaltenden Rückstand in die Pentensäurealkylestersynthesestufe a) zurückführt.

Das neue Verfahren hat den Vorteil, daß die Raum-Zeit-Ausbeute nicht nur erhalten bleibt, sondern sogar gesteigert wird. Zudem hat das neue Verfahren den Vorteil, daß ein hoher Umsatz an Butadien mit hoher Selektivität zu Pentensäurealkylestern erzielt wird. Ferner hat das neue Verfahren den Vorteil, daß die Bildung von Nebenprodukten nicht erhöht wird.

Als Ausgangsverbindung in Stufe a) verwendet man Butadien oder Butadien enthaltende Kohlenwasserstoffgemische. Vorteilhaft verwendet man Butadien enthaltende $C_4$-Schnitte. Solche $C_4$-Schnitte enthalten z.B. durchschnittlich 40 bis 60 Gew.% Butadien, 20 bis 35 Gew.% Isobuten, 10 bis 15 Gew.% Buten-1, 2 bis 15 Gew.% Buten-2 sowie 1 bis 10 Gew.% Butan.

Geeignete Alkanole haben vorteilhaft 1 bis 6 Kohlenstoffatome, insbesondere 1 bis 4 Kohlenstoffatome. Beispielhaft seien genannt: Methanol, Ethanol, Isopropanol, Butanol oder Hexanol. Besonders bevorzugt ist Methanol.

Die Alkanole wendet man in der Regel im Überschuß an. Vorteilhaft verwendet man je Mol Butadien 1,1 bis 10 Mol, insbesondere 1,5 bis 5 Mol Alkanole.

Die Umsetzung wird bei einer Temperatur von 80 bis 160°C, insbesondere 100 bis 150°C, durchgeführt. Ferner hält man bei der Umsetzung einen Druck von 100 bis 1000 bar, insbesondere von 120 bis 700 bar ein.

Kohlenmonoxid wird vorteilhaft im Überschuß, z.B. dem 1,3- bis 10-fachen der stöchiometrisch erforderlichen Menge angewandt. Vorteilhaft wird das überschüssige Kohlenmonoxid bei kontinuierlicher Arbeitsweise fortwährend im Kreis geführt und mit frischem Kohlenmonoxid ergänzt.

Die verwendeten Kobaltcarbonylkatalysatoren werden entweder in situ aus Kobaltsalzen, z.B. fettsauren Kobaltsalzen, wie Kobaltformiat, -acetat, -propionat oder -butyrat, erzeugt. Vorteilhaft bringt man den Katalysator bereits als Kobaltcarbonyl ein. Insbesondere hat es sich bewährt, wenn man Kobaltcarbonylkatalysator gelöst in Butadien oder $C_4$-Schnitt in das Reaktionsgemisch einbringt. Eine solche Lösung erhält man beispielsweise durch Umsetzen einer wäßrigen Lösung von fettsauren Kobaltsalzen mit einem Gemisch aus Kohlenmonoxid und Wasserstoff in Gegenwart von Aktivkohle bei einer Temperatur von 100 bis 175°C und unter einem Druck von 100 bis 400 bar. Aus der wäßrigen Lösung werden die entstandenen Kobaltcarbonylverbindungen dann mit Butadien oder Butadien enthaltenen Kohlenwasserstoffgemischen extrahiert.

Die Umsetzung wird in Gegenwart von heterocyclischen aromatischen tertiären Stickstoffbasen vorteilhaft mit einem $pK_A$-Wert von 6 bis 9 durchgeführt. Geeignete Stickstoffbasen sind beispielsweise 3-Methylpyridin ($pK_A$ 7,0), 4-Methylpyridin, 3,5-Dimethylpyridin, Chinolin oder Isochinolin. Es ist auch möglich, Gemische der genannten Stickstoffbasen anzuwenden. Besonders bevorzugt werden 3-Methylpyridin oder 4-Methylpyridin oder deren Gemische verwendet. Besonders bewährt hat es sich, wenn man je Mol Kobaltcarbonylkatalysator 2 bis 25 Mol der vorgenannten Stickstoffbasen anwendet.

Je Mol Butadien verwendet man vorteilhaft 0,01 bis 0,25 Mol Kobaltcarbonylkatalysator, insbesondere 0,04 bis 0,2 Mol Kobaltcarbonylkatalysator.

Man erhält ein Reaktionsgemisch, das Pentensäurealkylester, geringe Mengen $C_6$-Diester, tertiäre Stickstoffbasen, überschüssige Alkanole, nicht umgesetztes Butadien und gegebenenfalls weitere Kohlenwasserstoffe sowie nicht näher identifizierbare Nebenprodukte, wie polymeres Butadien, enthält. Das Reaktionsgemisch wird zweckmäßig entspannt und dann Pentensäuremethylester, Stickstoffbasen, überschüssige Alkanole und Kohlenwasserstoffe weitgehend abdestilliert (Stufe b). Die Destillation wird vorteilhaft bei einer Sumpftemperatur von 50 bis 55°C und unter einem Druck von 10 bis 20 mbar durchgeführt. Als Sumpfprodukt erhält man einen Kobaltkatalysator enthaltenden Rückstand, der neben dem Kobaltkatalysator noch Stickstoffbasen und Reste von Pentensäureestern, $C_6$-Diester sowie Hochsieder enthält. Ein typisches Gemisch enthält beispielsweise 9 bis 12 Gew.% Kobalt als Kobaltcarbonylkomplex, 25 bis 35 Gew.% Stickstoffbase und 3 bis 10 Gew.% Pentensäureester, 2 bis 6 Gew.% $C_6$-Diester sowie 45 bis 55 Gew.% hochsiedenden Rückstand.

In einer nachfolgenden Stufe c) wird der den Kobaltkatalysator enthaltende Rückstand bei erhöhter Temperatur und unter erhöhtem Druck mit einem Gemisch aus Kohlenmonoxid und Wasserstoff in Berührung gebracht. Vorteilhaft hält man hierbei eine Temperatur von 50 bis 200°C, insbesondere 100 bis 180°C und z.B. einen Druck von 10 bis 300 bar, insbesondere von 50 bis 200 bar ein.

Vorteilhaft verwendet man ein Gemisch aus 10 bis 90 Vol.-% Kohlenmonoxid und 10 bis 90 Vol.-% Wasserstoff. Es hat sich ferner bewährt, die Behandlung für einen Zeitraum von 5 bis 200, insbesondere 10 bis 150 Minuten durchzuführen. Anschließend wird das Gemisch aus Kohlenmonoxid und Wasserstoff abgetrennt.

Der so behandelte Katalysatorrückstand wird wiederum in die Pentenestersynthesestufe a) zurückgeführt.

Nach einer bevorzugten Arbeitsweise führt man einen Teil, z.B. 20 bis 90 Gew.%, des so behandelten Rückstands in die Pentenestersynthese zurück und ergänzt den vorhandenen Katalysator durch frischen Katalysator, während der restliche Teil, d.h. 10 bis 80 Gew.% als Katalysator für die Carbalkoxylierung von Pentensäureestern zu Adipinsäureestern verwendet wird. Dort wird nach Abtrennung der Wertprodukte der verbleibende Katalysator aufgearbeitet und somit auch Hochsieder entfernt. Dies hat den Vorteil, daß sich die Hochsieder im Katalysatorsystem nicht fortlaufend anreichern. Ein geeignetes Verfahren wird beispielsweise beschrieben in EP 10 581.

Das Verfahren nach der Erfindung sei an folgenden Beispielen veranschaulicht. Die angegebenen Gewichtsteile verhalten sich zu den Volumenteilen wie kg zu Liter.

Beispiel 1

In einem Hochdruckgefäß von 4 Vol.-Teilen führt man von unten im Verlauf von 60 Minuten 9,4 Gew.-Teile rückgeführten Katalysator aus der Carbalkoxylierung von Butadien zu Pentensäuremethylester, der durch Abtrennung der flüchtigen Komponenten erhalten wurde (10 % m/m) Kobalt als Kobaltcarbonylkomplex, 31 % (m/m) 3-Methylpyridin, 6 % (m/m) Pentensäuremethylester, 4 % (m/m) $C_6$-Diester, 48 % (m/m) Rückstand und 0,66 Gew.-Teile eines Gasgemisches aus 51 % (v/v) Kohlenmonoxid, 49 % (v/v) Wasserstoff zu. Die Aktivierung des Kobaltcarbonylkatalysators wurde bei 160°C und bei einem Druck von 100 bar durchgeführt. Nach dem Entspannen dieses Gemisches auf 1 bar wurden 26,4 Gew.-Teile $C_4$-Schnitt mit 41 % (m/m) Butadien-1,3, 19,5 Gew.-Teile 3-Methylpyridin, 8,8 Gew.-Teile Methanol und 21,6 Gew.-Teile eines Gasgemisches folgender Zusammenstzung: 83 Vol.-% Kohlenmonoxid, 1 Vol.-% Kohlendioxid, 3 Vol.-% Stickstoff, 0,07 Vol.-% Wasserstoff sowie 12 Vol.-% Butene zugefügt. Die Carbalkoxylierung verläuft bei einer Temperatur von 135°C und unter einem Druck von 650 bar in einem Hochdruckgefäß von 260 Vol.-Teilen. Das am Kopf des Hochdruckgefäßes abgenommene Produkt wird entspannt und die Gasphase abgetrennt. Anschließend werden die überschüssigen $C_4$-Kohlenwasserstoffe abdestilliert. Sie enthalten noch 1200 ppm nicht umgesetztes Butadien. Der Umsatz, bezogen auf Butadien, beträgt 99,8 %, die Selektivität zu Pentensäuremethylester beträgt 90 %. Die Raumzeitausbeute beträgt 0,079 kg Pentensäuremethylester je Liter Reaktionsraum und Stunde.

Vergleichsbeispiel

Man verfährt wie in Beispiel 1 beschrieben. Die Aktivierung des Kobaltcarbonylkatalysators wird jedoch bei Raumtemperatur durchgeführt und es werden nur 7,2 Gew.-Teile dieses Katalysatorgemisches mit 0,51 Gew.-Teilen des Kohlenmonoxid/Wasserstoff-Gemisches umgesetzt. Dieser Lösung werden nach der Entspannung auf 1 bar 20,2 Gew.-Teile C$_4$-Schnitt mit 41 % Butadien-1,3, 14,9 Gew.-Teile 3-Methylpyridin, 6,7 Gew.-Teile Methanol und 17,0 Gew.-Teile des obengenannten Gasgemisches zugegeben. Die Carbonylierung erfolgt wie in Beispiel 1 beschrieben. Der Umsatz, bezogen auf Butadien, beträgt 99,8 %, die Selektivität zu Pentensäuremethylester beträgt ebenfalls 90 %. Die Raumzeitausbeute beträgt 0,060 kg Pentensäuremethylester je Liter Reaktionsraum und Stunde.

**Patentansprüche**

1. Verfahren zur Herstellung von Pentensäurealkylestern, wobei man
   a) Butadien oder Butadien enthaltende Kohlenwasserstoffgemische mit Kohlenmonoxid und Alkanolen in Gegenwart von tertiären aromatischen heterocyclischen Stickstoffbasen und Kobaltcarbonylkatalysatoren bei einer Temperatur von 80 bis 160°C und unter einem Druck von 100 bis 1000 bar umsetzt und ein Pentensäurealkylester, C$_6$-Diester, tertiäre Stickstoffbasen, Alkanole, Butadien, Kohlenwasserstoffe und Nebenprodukte enthaltendes Reaktionsgemisch erhält
   b) aus dem erhaltenen Reaktionsgemisch überschüssige Kohlenwasserstoffe, Stickstoffbasen, Alkanole sowie Pentensäurealkylester durch Destillation weitgehend abtrennt und einen Kobaltcarbonylkatalysator enthaltenden Rückstand erhält
   c) den Kobaltcarbonylkatalysator enthaltenden Rückstand bei erhöhter Temperatur und unter erhöhtem Druck mit einem Gemisch aus Kohlenmonoxid und Wasserstoff in Berührung bringt und
   d) den Kobaltcarbonylkatalysator enthaltenden Rückstand in die Pentensäurealkylestersynthesestufe a) zurückführt.

2. Verfahren nach Anspruch 1, dadurch gekennzeichnet, daß man in der Stufe c) einen Druck von 10 bis 300 bar einhält.

3. Verfahren nach den Ansprüchen 1 und 2, dadurch gekennzeichnet, daß man in der Stufe c) eine Temperatur von 50 bis 200°C einhält.

4. Verfahren nach den Ansprüchen 1 bis 3, dadurch gekennzeichnet, daß man in Stufe c) ein Gemisch aus 10 bis 90 Vol.-% Kohlenmonoxid und 10 bis 90 Vol.-% Wasserstoff verwendet.

5. Verfahren nach den Ansprüchen 1 bis 4, dadurch gekennzeichnet, daß man in der Stufe c) eine Behandlungsdauer von 10 bis 150 Minuten einhält.

6. Verfahren nach den Ansprüchen 1 bis 5, dadurch gekennzeichnet, daß man einen Teil des Kobaltcarbonylkatalysators nach Inberührungbringen mit einem Gemisch aus Kohlenmonoxid und Wasserstoff in die Pentenestersynthese zurückführt und den verbleibenden Teil als Katalysator für die Carbalkoxylierung von Pentensäurealkylestern zu Adipinsäureestern verwendet.

**Claims**

1. A process for preparing an alkyl pentenoate by
   a) reacting butadiene or a butadiene-containing hydrocarbon mixture with carbon monoxide and an alkanol in the presence of a tertiary aromatic heterocyclic nitrogen base and a cobalt carbonyl catalyst at from 80 to 160°C under from 100 to 1,000 bar to obtain a reaction mixture which contains alkyl pentenoate, C$_6$-diester, tertiary nitrogen base, alkanol, butadiene, hydrocarbon and by-product,
   b) substantially removing from the reaction mixture obtained any excess hydrocarbon, nitrogen base, alkanol and alkyl pentenoate by distillation to obtain a residue which contains cobalt carbonyl catalyst,
   c) bringing the residue which contains cobalt carbonyl catalyst into contact with a mixture of carbon monoxide and hydrogen at elevated temperature and elevated pressure, and
   d) recycling the residue which contains cobalt carbonyl catalyst into alkyl pentenoate synthesis stage a).

2. A process as claimed in claim 1, wherein a pressure of from 10 to 300 bar is maintained in stage c).

3. A process as claimed in claims 1 and 2, wherein a temperature of from 50 to 200°C is maintained in stage c).

4. A process as claimed in claims 1 to 3, wherein a mixture of from 10 to 90% by volume of carbon monoxide and from 10 to 90% by volume of hydrogen is used in stage c).

5. A process as claimed in claims 1 to 4, wherein a treatment time of from 10 to 150 minutes is maintained in stage c).

6. A process as claimed in claims 1 to 5, wherein some of the cobalt carbonyl catalyst is contacted with a mixture of carbon monoxide and hydrogen and then recycled into the pentenoic ester synthesis while the remainder is used as a catalyst for the carbalkoxylation of alkyl pentenoates to adipic esters.

**Revendications**

1.- Procédé de préparation d'esters alkyliques d'acide penténoïque, dans lequel
a) on fait réagir du butadiène ou des mélanges d'hydrocarbures contenant du butadiène avec du monoxyde de carbone et des alcanols en présence de bases azotées hétérocycliques aromatiques tertiaires et de catalyseurs à base de cobalt-carbonyle, à une température de 80 à 160°C et sous une pression de 100 à 1000 bar, et on obtient un mélange réactionnel contenant des esters alkyliques d'acide penténoïque, des diesters en $C_6$, des bases azotées tertiaires, des alcanols, du butadiène, des hydrocarbures et des produits secondaires,
b) on sépare dans une large mesure par distillation, du mélange réactionnel obtenu, les hydrocarbures en excès, les bases azotées, les alcanols et les esters alkyliques d'acide penténoïque, et on obtient un résidu contenant le catalyseur à base de cobalt-carbonyle,
c) à température élevée et sous pression élevée, on met le résidu contenant le catalyseur à base de cobalt-carbonyle en contact avec un mélange de monoxyde de carbone et d'hydrogène, et
d) on renvoie le résidu contenant le catalyseur à base de cobalt-carbonyle dans l'étape a) de synthèse des esters alkyliques d'acide penténoïque.
2.- Procédé selon la revendication 1, caractérisé en ce qu'on maintient, dans l'étape c), une pression de 10 à 300 bar.
3.- Procédé selon la revendication 1 ou 2, caractérisé en ce qu'on maintient, dans l'étape c), une température de 50 à 200°C.
4.- Procédé selon l'une quelconque des revendications 1 à 3, caractérisé en ce qu'on utilise, dans l'étape c), un mélange de 10 à 90% en volume de monoxyde de carbone et de 10 à 90% en volume d'hydrogène.
5.- Procédé selon l'une quelconque des revendications 1 à 4, caractérisé en ce qu'on maintient, dans l'étape c), une durée de traitement de 10 à 150 mn.
6.- Procédé selon l'une quelconque des revendications 1 à 5, caractérisé en ce qu'après la mise en contact du catalyseur à base de cobalt-carbonyle avec un mélange de monoxyde de carbone et d'hydrogène, on en renvoie une partie dans la synthèse d'ester penténoïque et on utilise la partie restante comme catalyseur dans la carbalcoxylation d'esters allyliques d'acide penténoïque pour l'obtention d'esters d'acide adipique.